# EUROPEAN PATENT SPECIFICATION

(11) **EP 1 698 891 B1**
(45) Date of publication and mention of the grant of the patent: **05.09.2007**
(21) Application number: 05251284.5
(22) Date of filing: 03.03.2005
(51) Int. Cl.: G01N 27/49, C12Q 1/00

(54) **Method for reducing measuring bias in amperometric biosensors**
Methode zur Reduzierung der Messabweichung von amperometrischen Biosensoren
Méthode pour réduire la déviation de mesure d'un biocapteur ampérometrique

(43) Date of publication of application: 06.09.2006
(73) Proprietor: Apex Biotechnology Corporation, Hsinchu Science Park Hsinchu (TW)
(72) Inventor: Lin, Yueh-Hui 4F, No. 6, Innovation Road II, Hsinchu, Taiwan (CN); Huang, Ying-Che 4F, No. 6, Innovation Road II, Hsinchu, Taiwan (CN); Huang, Bin 4F, No. 6, Innovation Road II, Hsinchu, Taiwan (CN); Shen, Benjamin Cheng 4F, No. 6, Innovation, Hsinchu, Taiwan (CN); Chin, Li-Te 4F, No. 6, Innovation Road II, Hsinchu, Taiwan (CN); Shen, Thomas Y.S. 4F, No. 6, Innovation Road II, Hsinchu, Taiwan (CN)
(74) Representative: Nicholls, Michael John

(56) References cited:
- EP-A- 0 800 086
- EP-A- 1 236 995
- EP-A- 1 426 757
- EP-A- 1 447 452
- WO-A-01/21827

## Description

### FIELD OF THE INVENTION

The present invention relates to a method for reducing measuring bias in amperometric biosensors, characterized in that at the same time an analyte is applied to a sensor whose electrode system has been applied a predetermined potential, the background current resulted from the major interferences at the initial stage of the reaction of the biosensor is eliminated in the manner of gradually lowering the level of the predetermined potential.

### BACKGROUND OF THE INVENTION

Methods for measuring certain analytes in liquid test samples with amperometric biosensors are often used in monitoring of clinical physiological abnormalities, such as enzymatic amperometric blood sugar and lactate measuring methods, and non-enzymatic uric acid and hemoglobin measuring methods. Regardless of enzymatic or non-enzymatic system, an electric mediator is used as a transmitting mediator for electric signals of the redox reaction of the analyte.

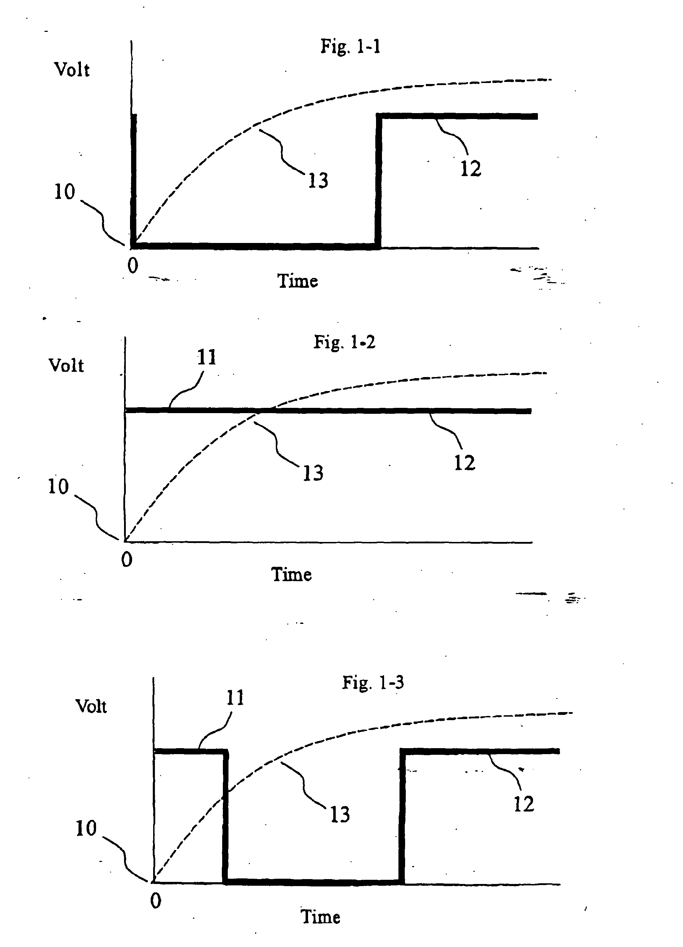

As indicated in Figure 1-1, since the analyte in the test sample carries out oxidation reaction, the trend of the electric mediator to be reduced (namely the electric signal is transmitted to the electric mediator) is gradually increased (13). After a sufficient reaction time, a fixed potential (12) is further applied to the electrode system, to enable the switching of electric signals between the electric mediator and the electrode, namely an electrochemical.redox reaction. Therefore, through the Cottrell current and concentration correlation as a result of the electrochemical redox reaction, namely the positive relativity of Cottrell current and the concentration of the analyte, the analyte concentration can be obtained by measuring the current of the working electrode (U.S. Pat. No. Re 36268).

During the storage period before being applied, the electric mediator or other components of the amperometric biosensor (for example, enzyme stabilizers or surfactants having oxidizing or reducing equivalents) may undergo oxidation or reduction reactions due to environmental influence (such as luminous energy, thermal energy or humidity). If the sensor containing reacted components is used to measure an analyte in a test sample, the reacted components will become background interference, and affect the current measuring value of the electrode system of the sensor, namely forming the bias value. Therefore, when using an amperometric biosensor, interference resulted from environmental influence must be reduced or eliminated so as to accurately determine the analyte concentration in the test sample.

At present, the common method for eliminating the background noise is to switch on the circuit of the electrode system and simultaneously eliminate the background interference, when the liquid test sample is applied to the sensor whose electrode system has been applied a predetermined potential, so as to reduce the bias value.

Figures 1-2 and 1-3 respectively illustrate two prior art potential operation modes. Figure 1-2 shows the potential operation mode of the blood sugar determining method disclosed in U.S. Pat. No. 4,224,125 (utilizing one of amperometric biosensors), in which at the same time (10) when a liquid test sample is applied to a sensor whose electrode system has been applied a predetermined potential (11), the steady current/concentration correlation is measured in a power-on manner Figure 1-3 shows the potential operation mode of the blood sugar determining method (utilizing one of amperometric biosensors) manufactured by Bayer (U.S. Pat. No. 5,653,836 and 5,620,579), in which at the same time (10) when the liquid test sample is applied to the sensor whose electrode system has been applied a predetermined potential, the fixed predetermined potential (11) is used to eliminate the background interference for a certain period of time, and then switched off. After a sufficient reaction time for the analyte in the test sample, a driving potential (12) is then re-applied to measure the current and concentration correlation.

However, while the former, which uses the power-on manner, can reduce the background current, it cannot accumulate the reaction intensity of the analyte in a short time (10 to 20 seconds) and thus reduce the signal intensity, but increases the power consumption. On the other hand, while the latter cuts off the power after using fixed potential to eliminate major background interferences so as to reduce the power consumption, it also eliminates the signal converted to the electric mediator through redox process at the initial stage after the analyte is applied to the measuring slot, and thus the signal during the earlier period of the reaction cannot be accumulated. As a result, in prior art operations, the economic efficiency and accuracy of the correlation between the current and the concentration of the analyte in the test sample remains to be improved.

The major object of this invention is to provide an improved method for reducing the measuring bias in amperometric biosensors.

### SUMMARY OF THE INVENTION

The present invention provides a novel method for reducing the measuring bias in amperometric biosensors, comprising the following steps:
(a) assembling an amperometric biosensor, which comprises at least an electrode system and an electric mediator for redox reactions, and applying a predetermined potential to said electrode system;
(b) applying a test sample to the biosensor and immediately gradually lowering the predetermined potential to a value unable to drive the electrochemical redox reaction of the electric mediator;
(c) after a sufficient reaction time, applying a second potential to the electrode system of the biosensor to drive the electrochemical redox reaction of the electric mediator; and
(d) measuring the electrochemical current of the electrode system and determining the concentration of the analyte.

The method for measuring the concentration of the analyte in the test sample provided by the present invention is characterized by, at the same time when the test sample is applied to the amperometric biosensor whose electrode system has been applied a predetermined potential to switch on the electrode system circuit, gradually lowering the predetermined potential to a value unable to drive a electrochemical redox reaction.

The method according to the invention not only can eliminate the background current of the major interference at the initial stage of the reaction, but also can retain the reacted electric mediator from the analyte in the test sample at the initial stage of the redox reaction as far as possible, and thus the electric signal at the initial stage of the reaction can be accumulated. The present invention not only can eliminate the interfering noise, but also can retain a high signal value as far as possible (namely, to increase the signal/noise (S/N) ratio), and thus improve the preciseness and accuracy of the correlation between the current and the concentration. In addition, since the predetermined potential supply is gradually lowered, when interrupting the electrochemical redox to await the signal accumulation of electric mediators, power consumption can also be reduced at the same time.

Persons skilled in the art is aware of that a digital or analog converting control and current signal capturing module can be used to control the potential supplied to the electrode system of the amperometric biosensor and to capture the current generated from the electrochemical redox reaction. Thus, the operation of gradually lowering potential in the invention can be performed in a digital or analog manner, depending on the type of the used converting control and current signal capturing module.

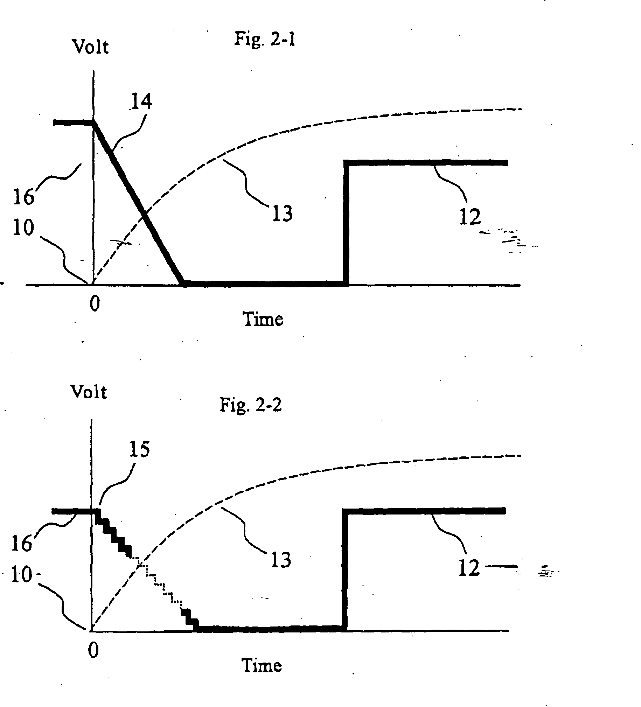

Figures 2-1 and 2-2 respectively illustrate the two modes of potential operation of the invention. Figure 2-1 shows the embodiment of the invention in which the potential is gradually lowered in an analog manner. First, a predetermined potential (16) is applied to the electrode system of an amperometric biosensor. Then, at the same time (10) when a liquid test sample is applied to the sensor, the potential is gradually lowered in an analog manner (14) while eliminating background interference. After a sufficient reaction time for the analyte in the test sample, a driving potential (12) is applied to measure the correlation between the current and the concentration.

Figure 2-2 shows the same potential operation mode as that shown in Figure 2-1, except that after the test sample has been applied to the electrode system of the sensor, the potential is gradually lowered in a digital manner (15).

The value of the predetermined potential applied to the electrode system of the sensor before the application of the test sample used in the method of the invention relates to the interfering degree of environmental factors, and generally ranges from about 0.1 volt to 0,9 volt, preferably from 0.3 volt to 0.7 volt, which is familiar to persons skilled in the art.

The step of gradually lowering the predetermined potential in the method according to the invention takes about 1 to 15 seconds, preferably from 4 to 10 seconds. The reaction time of the test sample and the value of the second potential used to drive the electric mediator to conduct a electrochemical redox reaction are not critical to the invention, and can be determined by persons skilled in the art according to the types of test samples and sensors actually used in the operation, wherein the second potential can be higher than, equal to, or lower than the predetermined potential.

The method according to the invention is applicable to enzymatic or non-enzymatic amperometric biosensors. The structures of enzymatic or non-enzymatic amperometric biosensors are familiar to persons skilled in the art. The method according to the invention is not limited to any particular type of amperometric biosensor. Generally, the enzymatic amperometric biosensor typically comprises an electrode system, a redox enzyme specific to particular analyses, an electric mediator for redox, a carrier and buffering agent consisted of conjugate acid and base; and the non-enzymatic amperometric biosensor typically comprises an electrode system, an electric mediator for redox and a carrier. The carrier generally includes water-soluble high polymers, and optionally includes surfactants. The electric mediator for redox is usually potassium ferricyanide.

### BRIEF DESCRIPTION OF THE DRAWINGS

Figures 1-1, 1-2 and 1-3 show three potential operation modes in the art.

Figure 2 shows the operation mode of gradually lowering the potential according to the invention, wherein Figure 2-1 shows the gradual lowering of potential in an analog manner, while Figure 2-2 shows the gradual lowering of potential in a digital manner.

Figure 3 shows the improvement in the analytic degree of the correlation between the current and the concentration of glucose measured with the method according to the invention.

### DETAILED DESCRIPTION OF THE INVENTION

The glucose biosensor test strip available from the implementation of ROC (Taiwan) Pat. No. 416005 is used to further show the advantages of the method according to the invention.

The glucose biosensor test strip is of the structural components and combination as shown in Figure 1 and Figure 2 thereof, and is of the post-modification formulation of the glucose reaction layer illustrated in Example 1 thereof The formulation includes a glucose oxidase, potassium ferricyanide, a surfactant, a water-soluble high polymer and a buffering salt.

The environmental interference is increased by the accelerated aging stimulation (storage environment for the test strip being at 50°C, lasting for 1 week and eight weeks, respectively). A digital converting control and current signal capturing module is used to control the potential supplied to the electrode system of the amperometric glucose biosensor and to capture the current generated from the electrochemical redox reaction. Finally, the acquired current reading is converted into the glucose concentration.

The following examples are used to provide further illustration for the invention, but not intended to restrict the scope of the invention. All those skilled in the art can easily make modifications and changes without departing from the scope of the invention.

### Example

Two groups of power supply modes were set, one as the initial supply potential of 300 mV, which was reduced to 0 mV immediately after the test sample was applied to the electrode system as used in prior art (Figure 1-1), the other as the initial supply potential of 300 mV, which was gradually lowered to 0 mV 4 seconds after the test sample was applied to the electrode system (i.e., the electrochemical redox reaction being interrupted) according to the conception of the invention (Figure 2-2).

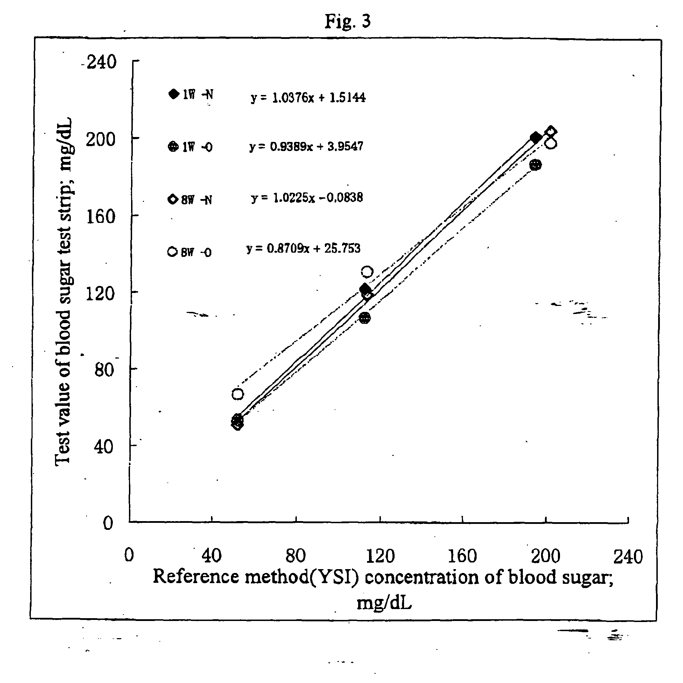

The above-mentioned two groups of power supply setting modes and bloods with different concentration of glucose (lower, medium, and higher) were applied to the same batch of glucose biosensor test stripes being accelerated aging processed. The reaction was maintained for 16 seconds. Then, a driving potential of 300mV was applied, and the redox current in positive correlation with the concentration of glucose was measured. The result is shown in Figure 3.

When applying the prior art power supply mode to the glucose biosensor test strip, the intercept of the linear equation drawn with the sensor reading (lasting one week = 1W-O, and lasting 8 weeks = 8W-O) and reference method (YSI electrochemistry blood sugar biochemical meter) was increased (from 3.95 to 25.75), which shows the obvious existence of background interference generated from the acceleration condition. However, when applying the power supply mode according to the conception of the invention to the glucose biosensor test strip (lasting 1 week = 1W-N, and lasting 8 weeks = 8W-N), the intercept of the linear equation shows no significant difference (1.51 and -0.08), which shows the apparent reduction of background interference.

Furthermore, the method according to the invention adopts the mode of gradually lowering the supplied potential to 0 mV, which interrupts the electrochemical redox reaction. Therefore, during the period awaiting the signal accumulation of electric mediator, power consumption can be reduced. In conclusion, the method according to the invention is in fact an effective method to reduce the latent bias value caused by background interference for amperometric biosensors.

## Claims

1. A method for determining the concentration of an analyte in a test sample, comprising the following steps:
(a) assembling an amperometric biosensor which comprises at least an electrode system and an electric mediator for redox reactions, and applying a predetermined potential to the electrode system;
(b) applying a test sample to the biosensor and immediately gradually lowering the predetermined potential to a value unable to drive the electrochemical redox reaction of the electric mediator;
(c) after a sufficient reaction time, applying a second potential to the electrode system of the biosensor to drive the electrochemical redox reaction of the electric mediator; and
(d) measuring the electrochemical current of the electrode system and determining the concentration of the analyte.

2. The method of Claim 1, wherein said step of gradually lowering the predetermined potential is performed in a digital manner.

3. The method of Claim 1, wherein said step of gradually lowering the predetermined potential is performed in an analog manner.

4. The method of Claim 1, wherein said sensor is an enzymatic amperometric biosensor.

5. The method of Claim 1, wherein said sensor is a non-enzymatic amperometric biosensor.

6. The method of any of Claims 1 to 5, wherein said predetermined potential is within the range from 0.1 volt to 0.9 volt.

7. The method of Claim 6, wherein said predetermined potential is within the range from 0.3 volt to 0.7 volt.

8. The method of any of Claims 1 to 5, wherein said step of gradually lowering the predetermined potential takes one to fifteen seconds.

9. The method of Claim 8 wherein said step of gradually lowering the predetermined potential takes four to ten seconds.

10. The method any of Claims 1 to 5, wherein said second potential can be higher than, equal to or lower than the predetermined potential.

## Patentansprüche

1. Verfahren zur Bestimmung der Konzentration eines Analyten in einer Testprobe, umfassend die folgenden Stufen:
(a) das Zusammensetzen eines amperometrischen Biosensors, der mindestens ein Elektrodensystem und einen elektrischen Mediator für Redoxreaktionen umfasst, und das Anlegen eines vorgegebenen Potentials an das Elektrodensystem;
(b) das Aufbringen einer Testprobe auf den Biosensor und das sofortige allmähliche Absenken des vorgegebenen Potentials auf einen Wert, der nicht befähigt ist, die elektrochemische Redoxreaktion des elektrischen Mediators anzutreiben;
(c) nach Ablauf einer ausreichenden Reaktionszeit das Anlegen eines zweiten Potentials an das Elektrodensystem des Biosensors, um die elektrochemische Redoxreaktion des elektrischen Mediators anzutreiben; und
(d) das Messen des elektrochemischen Stroms des Elektrodensystems und das Bestimmen der Konzentration des Analyten.

2. Verfahren nach Anspruch 1, wobei die Stufe des allmählichen Absenkens des vorgegebenen Potentials in digitaler Weise durchgeführt wird.

3. Verfahren nach Anspruch 1, wobei die Stufe des allmählichen Absenkens des vorgegebenen Potentials in analoger Weise durchgeführt wird.

4. Verfahren nach Anspruch 1, wobei es sich beim Sensor um einen enzymatischen amperometrischen Biosensor handelt.

5. Verfahren nach Anspruch 1, wobei es sich beim Sensor um einen nicht-enzymatischen amperometrischen Biosensor handelt.

6. Verfahren nach einem der Ansprüche 1 bis 5, wobei das vorgegebene Potential im Bereich von 0,1 Volt bis 0,9 Volt liegt.

7. Verfahren nach Anspruch 6, wobei das vorgegebene Potential im Bereich von 0,3 Volt bis 0,7 Volt liegt.

8. Verfahren nach einem der Ansprüche 1 bis 5, wobei die Stufe des allmählichen Absenkens des vorgegebenen Potentials 1 bis 15 Sekunden dauert.

9. Verfahren nach Anspruch 8, wobei die Stufe des allmählichen Absenkens des vorgegebenen Potentials 4 bis 10 Sekunden dauert.

10. Verfahren nach einem der Ansprüche 1 bis 5, wobei das zweite Potential höher, gleich groß oder niedriger als das vorgegebene Potential sein kann.

## Revendications

1. Procédé pour déterminer la concentration d'un analyte dans un échantillon test, comportant les étapes suivantes :
(a) assembler un biocapteur ampérométrique qui comporte au moins un système d'électrode et un médiateur électrique pour des réactions redox, et appliquer un potentiel prédéterminé au système d'électrode ;
(b) appliquer un échantillon test au biocapteur et abaisser immédiatement graduellement le potentiel prédéterminé à une valeur incapable d'entraîner la réaction redox électrochimique du médiateur électrique ;
(c) après un temps de réaction suffisant, appliquer un second potentiel au système d'électrode du biocapteur pour entraîner la réaction redox électrochimique du médiateur électrique ; et
(d) mesurer le courant électrochimique du système d'électrode et déterminer la concentration de l'analyte.

2. Procédé selon la revendication 1, dans lequel ladite étape consistant à abaisser graduellement le potentiel prédéterminé est effectuée d'une manière numérique.

3. Procédé selon la revendication 1, dans lequel ladite étape consistant à abaisser graduellement le potentiel prédéterminé est effectuée d'une manière analogique.

4. Procédé selon la revendication 1, dans lequel ledit capteur est un biocapteur ampérométrique enzymatique.

5. Procédé selon la revendication 1, dans lequel ledit capteur est un biocapteur ampérométrique non-enzymatique.

6. Procédé selon l'une quelconque des revendications 1 à 5, dans lequel ledit potentiel prédéterminé se trouve dans la plage de 0,1 volt à 0,9 volt.

7. Procédé selon la revendication 6, dans lequel ledit potentiel prédéterminé se trouve dans la plage de 0,3 volt à 0,7 volt.

8. Procédé selon l'une quelconque des revendications 1 à 5, dans lequel ladite étape consistant à abaisser graduellement le potentiel prédéterminé prend de une à quinze secondes.

9. Procédé selon la revendication 8, dans lequel ladite étape consistant à abaisser graduellement le potentiel prédéterminé prend de quatre à dix secondes.

10. Procédé selon l'une quelconque des revendications 1 à 5, dans lequel ledit second potentiel peut être supérieur, égal ou inférieur au potentiel prédéterminé.
